# EUROPEAN PATENT SPECIFICATION

(11) **EP 2 558 440 B1**
(45) Date of publication and mention of the grant of the patent: **16.11.2016**
(21) Application number: 11714311.5
(22) Date of filing: 14.04.2011
(51) Int. Cl.: C07C 231/24, C07C 233/18, A61K 31/165, A61P 25/24, A61P 3/10

(54) **PROCESS FOR THE PRODUCTION OF POLYMORPH FORM I OF AGOMELATINE**
VERFAHREN ZUR HERSTELLUNG EINER POLYMORPHEN FORM I VON AGOMELATIN
PROCÉDÉ POUR LA PRODUCTION DE LA FORME POLYMORPHE I D'AGOMÉLATINE

(30) Priority: 15.04.2010 EP 10159998
(43) Date of publication of application: 20.02.2013
(73) Proprietor: ratiopharm GmbH, 89079 Ulm (DE)
(72) Inventor: FISCHER, Dirk, 89073 Ulm (DE); MATZDORF, Thorsten, 89233 Neu-Ulm (DE); NESS, Winfried, 89081 Ulm (DE)
(74) Representative: D Young & Co LLP
(86) International application number: PCT/EP2011/055935
(87) International publication number: WO 2011/128413

(56) References cited:
- US-A1- 2008 280 991
- TINANT B ET AL: "N-Ä2-(7-METHOXY-1-NAPHTHYL)ETHYLACETAMIDE , A POTENT MELATONIN ANALOG", ACTA CRYSTALLOGRAPHICA SECTION C. CRYSTAL STRUCTURE COMMUNICATIONS, MUNKSGAARD, COPENHAGEN, DK LNKD- DOI:10.1107/S0108270193012922, vol. C50, no. 6, 1 January 1994 (1994-01-01), pages 907-910, XP009047983, ISSN: 0108-2701 cited in the application
- YOUS S ET AL: "NOVEL NAPHTHALENIC LIGANDS WITH HIGH AFFINITY FOR THE MELATIONIN RECEPTOR", JOURNAL OF MEDICINAL CHEMISTRY, AMERICAN CHEMICAL SOCIETY, WASHINGTON, US LNKD- DOI:10.1021/JM00086A018, vol. 35, no. 8, 17 April 1992 (1992-04-17) , pages 1484-1486, XP002016145, ISSN: 0022-2623 cited in the application

## Description

The present invention relates to a process for preparing polymorph form I of agomelatine and a process for preparing medicaments with the polymorph form I of agomelatine, which includes the process for the preparation of the polymorph form I of agomelatine. The present invention also relates to the polymorph form I of agomelatine, which is obtainable by the new process and medicaments containing this polymorph form I of agomelatine.

Melatonin is a neurohormone that is physiologically synthesized principally in the pineal gland and is involved in the regulation of many physiological and pathophysiological processes such as sleep, seasonal disorders, depression and ageing. Melatonin exhibits its physiological actions by activating G-protein-coupled melatonin receptors MT1 and MT2.

Due to its involvement in several physiological and pathophysiological processes melatonin was considered as a possible therapeutically usable substance. However, the therapeutical usage of melatonin is limited by the fast metabolic degradation. The plasma half-life of melatonin is only about 15 minutes, which strongly restricts its therapeutical usability.

Research efforts have been made for structurally modified melatonin analogues which maintain the pharmacological activity of melatonin but which have improved pharmacokinetic properties. As a result of this research the bioisosterically modified melatonin analogue agomelatine attracted attention as a suitable therapeutic substance. Agomelatine has the chemical name N-[2-(7-methoxy-1-naphthyl)ethyl]acetamide and has the following chemical structure:

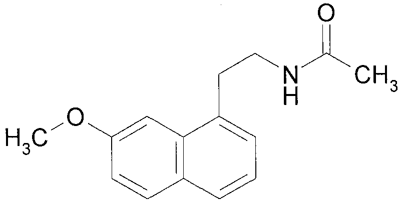

Agomelatine, its preparation and use have first been described in EP-A 0 447 285. It is a pharmacological agent with a dual mechanism of action. Similar to melatonin, agomelatine is an agonist of the melatonergic MT1 and MT2 receptors and, additionally, an antagonist of the 5-HT2c receptors. In the following agomelatine has been extensively investigated.

S. Yous et al. in J. Med. Chem. 1992, 35, 1484-1486 describe a process for the preparation of agomelatine, wherein agomelatine is obtained from a biphasic medium of water and chloroform. Bernard Tinant and Jean-Paul Declercq in Acta Cryst. (1994), C50, 907-910 disclose a full crystallographic investigation of the agomelatine produced by Yous et al. The polymorph form of the product obtained by Yous et al. and analyzed by Tinant and Declercq is designated as the polymorph form I of agomelatine. Tinant and Declercq provide the full crystal data of this polymorph form, and regarding the identification of the polymorph form I of agomelatine, it is explicitly referred to Bernard Tinant and Jean-Paul Declercq in Acta Cryst. (1994), C50, 907-910. The most important crystal data is as follows:

C₁₅H₁₇NO₂

Mr = 243.30
Orthorhombic

Pca2₁

a = 31.501 (4) A
b = 9.5280 (10) A
c = 17.906 (2) Å.

The density is D = 1203 kg • m⁻³, and the number of molecules in one cell is Z = 16.

Tinant and Declercq also disclose the fractional atomic coordinates and equivalent isotropic displacement parameters and selected geometric parameters as well as the hydrogen bonding geometry of this polymorph form. This data is included herein by reference.

EP 0 447 285 in example 1 also discloses a process for the preparation of agomelatine. In this process agomelatine is obtained by recrystallization from isopropyl ether. No information on the polymorph form can be found in EP 0 447 285.

Later publications consider that the agomelatine obtained according to EP 0 447 285 is the same polymorph form obtained by Yous et al. (e.g. US 2005/0182276) but no experimental data is provided.

The known processes for obtaining the polymorph form I of agomelatine are considered as providing the polymorph form I of agomelatine not in a sufficiently reproducible and well-defined manner and with only poor filtrability (US 2005/0182276).

Furthermore, the polymorph form I of agomelatine obtained by the known processes tends to have a relatively high hygroscopicity, which is considered a disadvantage. It would be useful to have a process which produces agomelatine of form I with a low hygroscopicity.

Finally, the processes for producing agomelatine of polymorph form I of the prior art usually provide a very broad particle size distribution for the agomelatine. It would be useful to have a process which produces agomelatine of polymorph form I with a narrower particle size distribution. This would facilitate the preparation of pharmaceutical formulations with the agomelatine of form I.

Several processes have been developed to prepare agomelatine crystals to overcome the problems of the prior art processes. However, none of these processes produced the polymorph form I of agomelatine, but different new polymorph forms of agomelatine were obtained. In particular, an attempt to dissolve agomelatine in a water-soluble solvent that is essentially acceptable in pharmaceutical compositions, such as ethanol, and crystallizing the agomelatine by adding water to the solution resulted in a new polymorph form which was designated polymorph form II of agomelatine. This polymorph form II has a monoclinic crystal lattice and not an orthorhombic crystal lattice and a different space group (namely P2₁/N). Form II has eight molecules in the unit cell and not 16 as the polymorph form I of agomelatine and has lattice parameters A = 20.0903 Å, B = 9.3194 Å, and C = 15.4796 A. This polymorph form (polymorph form II) of agomelatine is described e.g. in EP-A 1 564 202 and US 2005/0182276. In the process developed in these documents the agomelatine is prepared in ethanol, and water is added to the hot ethanol to precipitate the agomelatine.

Other processes are also known for the preparation of crystal forms of agomelatine, but those processes lead to other polymorph forms of agomelatine. In particular, US 2009/0069434 discloses a process which leads to crystalline form VI of agomelatine, US 2006/0270876 discloses a process which leads to a polymorph form III of agomelatine, US 2006/0270877 discloses a process which leads to the polymorph form V of agomelatine, and US 2006/0270875 discloses a process which leads to the polymorph form IV of agomelatine.

A polymorph form of agomelatine for use in a medicament should have excellent properties, such as crystallinity, polymorph stability, chemical stability and processability to pharmaceutical compositions.

Therefore, there exists a need in the art for a process for reliably and reproducibly producing the polymorph form I of agomelatine with good filtrability and in a very high chemical and polymorph purity. The obtained polymorph form I of agomelatine ideally should have a very low hygroscopicity, and the agomelatine of form I should be obtained in crystals with a narrow particle size distribution.

Unexpectedly it was found that the polymorph form I of agomelatine can be reliably obtained by an easy process in excellent reproducibility using a wide variety of different solvents, if certain process conditions are maintained. The obtained polymorph form I of agomelatine has a chemical purity of preferably 98% or more, more preferably 99% or more, in particular 99.5% or more, such as 99.7% or more. The obtained polymorph form I of agomelatine has a polymorph purity of preferably 98% or more, more preferred 99% or more, in particular 99.5% or more, such as 99.7% or more (all %-values in this context are % by weight). The obtained polymorph from I of agomelatine preferably has a low hygroscopicity, and with the process of the invention agomelatine of polymorph form I is obtained in the form of crystals having a very narrow particle size distribution.

The present invention provides a process for the preparation of the polymorph form I of agomelatine, characterized by the following crystal data:

C₁₅H₁₇NO₂

Mr = 243.30
Orthorhombic

Pca2₁

a = 31.501 (4) A
b = 9.5280 (10) A
c = 17.906 (2) A
Z= 16
the process comprising the process steps of:
a) dissolving agomelatine in a solvent to obtain a solution,
b) adding the solution to an organic anti-solvent having a temperature of 30°C or below, whereby crystals of the polymorph form I of agomelatine are formed, wherein the solvent of step a) is miscible with the anti-solvent of step b),
c) isolating the crystals of the polymorph form I of agomelatine and
d) optionally drying the isolated crystals of the polymorph form I of agomelatine.

The present invention also provides a process for the preparation of a medicament containing as active ingredient the polymorph form I of agomelatine as defined in claim 1, comprising the following steps:
i) carrying out the process of the invention in order to obtain the polymorph form I of agomelatine as defined in claim 1 and
ii) mixing the polymorph form I of agomelatine obtained in step i) with one or more pharmaceutically acceptable excipients.

The agomelatine obtained by the process of the present invention is of the known polymorph form I. However, the crystals obtained by the process of the present invention have a very narrow particle size distribution. The processes of the prior art yield agomelatine crystals of polymorph form I with a broader particle size distribution. Therefore, polymorph form I of agomelatine obtained by the process of the present invention is distinguishable from and thus novel over the products obtained by the prior art processes.

Furthermore, the crystals of polymorph form I of agomelatine obtained by the process of the present invention have a specific surface and are therefore less hygroscopic than the crystals of polymorph form I of agomelatine obtained by other processes.

The present invention also relates to those novel products and to pharmaceutical compositions containing the novel compounds.

The agomelatine of polymorph form I obtained by the process of the present invention has the required excellent properties regarding crystallinity, polymorph stability, chemical stability, filtrability and processability to pharmaceutical compositions and a high chemical and polymorph purity, as indicated above. It also has a very low hygroscopicity and is obtained in the form of crystals with a narrow particle size distribution.

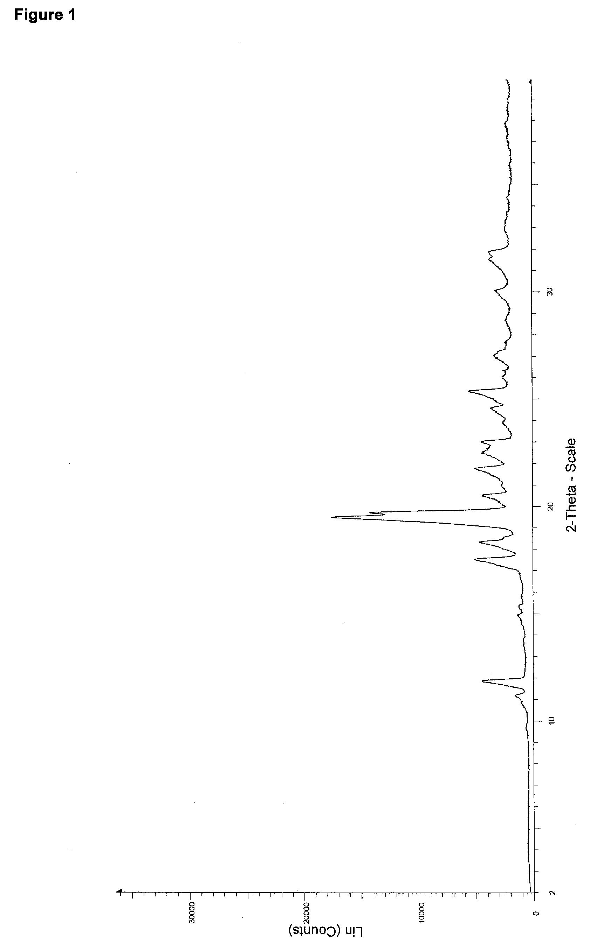

The agomelatine obtained by the process of the present invention is the polymorph form I of agomelatine which is known from Acta Cryst. (1994), C50, 907-910. Regarding the details and the definition of the crystal form, it is referred to this document. The polymorph form I of agomelatine can also be characterized by its powder diffraction diagram. An XRPD of agomelatine of form I that has been obtained by the process of example 1 of the present application is enclosed as figure 1. The XRPD corresponds to the theoretical XRPD that can be calculated on the basis of the full crystallographic analysis disclosed in Acta Cryst. (1994), C50, 907-910. The following table provides the relevant data of the powder diffractogram:

| Caption | Angle 2-Theta ° | d value Angstrom | Intensity Count | Intensity % |
|---|---|---|---|---|
| d=9,16671 | 9,641 | 9,16671 | 592 | 3,4 |
| d=8,17231 | 10,817 | 8,17231 | 1029 | 5,9 |
| d=7,94787 | 11,124 | 7,94787 | 1538 | 8,7 |
| d=7,48822 | 11,809 | 7,48822 | 4400 | 25 |
| d=6,43980 | 13,74 | 6,4398 | 784 | 4,5 |
| d=6,08725 | 14,54 | 6,08725 | 986 | 5,6 |
| d=5,94977 | 14,878 | 5,94977 | 1346 | 7,7 |
| d=5,79017 | 15,29 | 5,79017 | 1179 | 6,7 |
| d=5,61622 | 15,767 | 5,61622 | 1007 | 5,7 |
| d=5,06951 | 17,48 | 5,06951 | 5004 | 28,5 |
| d=4,84611 | 18,292 | 4,84611 | 4585 | 26,1 |
| d=4,55400 | 19,477 | 4,554 | 17581 | 100 |
| d=4,50686 | 19,682 | 4,50686 | 14226 | 80,9 |
| d=4,33285 | 20,481 | 4,33285 | 4285 | 24,4 |
| d=4,23516 | 20,959 | 4,23516 | 2627 | 14,9 |
| d=4,08651 | 21,73 | 4,08651 | 4987 | 28,4 |
| d=3,94971 | 22,493 | 3,94971 | 4308 | 24,5 |
| d=3,86781 | 22,975 | 3,86781 | 4349 | 24,7 |
| d=3,72287 | 23,883 | 3,72287 | 2487 | 14,1 |
| d=3,62328 | 24,549 | 3,62328 | 3605 | 20,5 |
| d=3,51028 | 25,352 | 3,51028 | 5519 | 31,4 |
| d=3,42274 | 26,012 | 3,42274 | 2551 | 14,5 |
| d=3,38527 | 26,305 | 3,38527 | 2267 | 12,9 |
| d=3,29485 | 27,041 | 3,29485 | 3303 | 18,8 |
| d=3,22416 | 27,645 | 3,22416 | 2375 | 13,5 |
| d=3,11066 | 28,675 | 3,11066 | 2269 | 12,9 |
| d=2,99868 | 29,77 | 2,99868 | 2641 | 15 |
| d=2,97275 | 30,036 | 2,97275 | 3070 | 17,5 |
| d=2,83475 | 31,535 | 2,83475 | 3689 | 21 |
| d=2,80725 | 31,852 | 2,80725 | 3667 | 20,9 |
| d=2,71617 | 32,95 | 2,71617 | 2296 | 13,1 |
| d=2,68998 | 33,28 | 2,68998 | 2252 | 12,8 |
| d=2,60507 | 34,398 | 2,60507 | 2107 | 12 |
| d=2,49650 | 35,944 | 2,4965 | 1887 | 10,7 |
| d=2,44059 | 36,797 | 2,44059 | 1923 | 10,9 |
| d=2,41420 | 37,213 | 2,4142 | 2138 | 12,2 |
| d=2,37420 | 37,864 | 2,3742 | 2279 | 13 |
| d=7,48822 | 11,809 | 7,48822 | 4400 | 25 |
| d=5,06951 | 17,48 | 5,06951 | 5004 | 28,5 |
| d=4,84611 | 18,292 | 4,84611 | 4585 | 26,1 |
| d=4,55400 | 19,477 | 4,554 | 17581 | 100 |
| d=4,50686 | 19,682 | 4,50686 | 14226 | 80,9 |
| d=4,33285 | 20,481 | 4,33285 | 4285 | 24,4 |
| d=4,08651 | 21,73 | 4,08651 | 4987 | 28,4 |
| d=3,94971 | 22,493 | 3,94971 | 4308 | 24,5 |
| d=3,86781 | 22,975 | 3,86781 | 4349 | 24,7 |
| d=3,62328 | 24,549 | 3,62328 | 3605 | 20,5 |
| d=3,51028 | 25,352 | 3,51028 | 5519 | 31,4 |
| d=3,29485 | 27,041 | 3,29485 | 3303 | 18,8 |
| d=2,97275 | 30,036 | 2,97275 | 3070 | 17,5 |
| d=2,83475 | 31,535 | 2,83475 | 3689 | 21 |
| d=2,80725 | 31,852 | 2,80725 | 3667 | 20,9 |

The polymorph form I of agomelatine is thus also defined by the above X-ray powder diffractogram, in particular by the 2-Theta values of the peaks having an intensity of at least 30%, more preferred of the peaks having an intensity of at least 20%, most preferred of the complete diffractogram. The error margin of the 2-Theta values is ± 0.2.

For the process of the present invention it is important that the agomelatine is dissolved in a suitable solvent to obtain a solution, and the solution is then added to an organic anti-solvent. In the prior art processes are disclosed in which agomelatine was dissolved in a water-miscible solvent, and then water was added to the solution. However, these processes did not produce agomelatine of form I but agomelatine of form II (US 2005/0182276, example 1).

For the process of the present invention it is important that the solution with the dissolved agomelatine is added to the organic anti-solvent having a low temperature of not more than 30°C, preferably not more than 20°C, most preferred not more than 10°C. In a particularly preferred embodiment the solution of the agomelatine is added to an organic anti-solvent with a temperature of -5°C to 5°C or even lower than -5°C. Preferred is also a temperature range of -5°C to 78°C, more preferred of -10°C to -78°C. Of course, the temperature of the organic anti-solvent must be so high that the anti-solvent is in liquid form. The temperature should also be so high that the solvent of step a) of the present invention is in liquid form, because otherwise the solvent solidifies when the solution with the agomelatine is added to the anti-solvent, which would be highly undesirable.

If the solution of agomelatine in a solvent is added to the organic anti-solvent having a too high temperature, mixtures of different polymorph forms of agomelatine are obtained.

The agomelatine that is used in the process of the present invention for preparing the polymorph I of agomelatine can have been prepared by any method known in the art, and it can be referred e.g. to the basic patent EP 0 447 285 or any of the other patents mentioned above which disclose processes for preparing agomelatine. Particularly preferred is that the agomelatine is prepared following the process disclosed in Yous et al. as mentioned above, but not in the biphasic (H₂O-CHCl₃) medium but in a solvent that is then subjected to the further process steps of the present invention. Of course, the process of Yous et al. can be strictly followed, and then the process of the present invention is carried out in order to obtain the advantageous polymorph form I of agomelatine with low hygroscopicity. Similarly, any known process for preparing agomelatine can be used for preparing the polymorph form I of agomelatine according to the present invention by either selecting an appropriate solvent for the last reaction step (if the obtained product already has the required purity) or by carrying out the process of the present invention as an additional reaction step.

In the process of the present invention in a first step agomelatine is dissolved in a suitable solvent. The solvent is not particularly restricted, but it is preferred that the solvent is pharmaceutically acceptable. The solvent can be a protic or an aprotic solvent. Suitable protic solvents comprise primary, secondary and tertiary alcohols with a straight or branched, cyclic or acyclic alkyl chain with one to six carbon atoms, carbon acids, carbon acid amides, primary, secondary and tertiary amines with a straight or branched, cyclic or acyclic alkyl chain with one to six carbon atoms. Preferred protic solvents comprise methanol, ethanol, propan-2-ol, acetic acid and propylamine.

In one embodiment of the invention the solvent is an aprotic solvent, and suitable aprotic solvents comprise aldehydes, ketones, ethers, esters, sulfoxides and carbon acid amides. Preferred aprotic solvents are acetone, 1,4-dioxane, dimethylsulfoxide, N-methylpyrrolidone, tetrahydrofuran (THF) and ethylacetate.

Particularly preferred are methanol, ethanol, propan-2-ol, acetone, tetrahydrofuran and ethylacetate.

The temperature at which the agomelatine is dissolved in the solvent is not particularly restricted, but usually the temperature is room temperature or lower. Most preferably the agomelatine is dissolved in the solvent at the temperature in which step b) is performed afterwards. Generally, the solvent for agomelatine can be any organic solvent in which the agomelatine at the temperature at which step b) of the process of the invention is performed is better soluble than in the anti-solvent.

The anti-solvent used in step b) of the process of the invention can be any organic solvent in which agomelatine at the temperature at which step b) of the process of the invention is performed is less soluble than in the solvent, so that the agomelatine crystallizes when it is added to the anti-solvent. According to the invention it is important that the solvent of step
a) of the process of the invention is miscible with the anti-solvent of step b) of the process of the invention. A solvent is miscible with the anti-solvent, if, at the temperature at which step
b) of the present invention is carried out, at the end of step b) solvent and anti-solvent form a homogeneous solution. Preferably, the solvent is miscible with the anti-solvent, resulting in a homogeneous solution at a broad range of solvent/anti-solvent ratios from 1:100 to 1:1 (v/v), preferably at ratios from 1:20 to 1:2 (v/v) at the temperature at which step b) is performed.

Preferred anti-solvents are organic unpolar compounds that are liquid at the temperature at which step b) of the process of the invention is performed. Most preferred anti-solvents are petrolether, and all petrolether fractions can be used, particularly preferred is the low polybenzene fraction with a boiling temperature in the range of 25 to 80°C and a density in the range of 0.63 to 0.83.

Furthermore preferred are alkanes, and all alkanes can be used which are liquid at the temperature at which step b) of the process of the invention is performed. Most preferred are alkanes with five to eight carbon atoms, and the most preferred alkanes are n-hexane and n-heptane, most preferred is n-heptane.

With the above unpolar anti-solvents the process of the present invention can be carried out at relatively high temperatures in the range of 0°C to room temperature and with those unpolar anti-solvents the preferred temperature range for carrying out step b) of the process of the present invention is between -5°C to 5°C.

Other suitable anti-solvents are e.g. diisopropylether, glycerol and triethylamine, however, with those anti-solvents the process of step b) should be carried out at lower temperatures, in particular at temperatures of less than 0°C, more preferably of less than -5°C and most preferably at temperatures of -10°C or below.

With all anti-solvents useful in the process of the present invention the lowest possible temperature for carrying out process step b) of the process of the present invention is the melting point of the anti-solvent, because in step b) of the process of the present invention, of course, the anti-solvent must be liquid.

Furthermore, of course, the temperature for carrying out step b) of the process of the present invention should be higher than the melting point of the solvent in which the agomelatine is dissolved, because otherwise this solvent would freeze when it is added to the anti-solvent, which would complicate or prevent the crystallization of the agomelatine of polymorph form I.

According to the invention it was also unexpectedly found that a very high reproducibility of the product characteristics and a very good filtrability can be obtained by using a sufficiently high excess of organic anti-solvent. Preferably the solvent/anti-solvent ratio in process step b) of the process of the present invention is at least 1:2, more preferably at least 1:3 and most preferably at least 1:4.

The amount of anti-solvent most preferably is significantly higher than the amount of solvent for agomelatine, and a solvent/anti-solvent ratio of 1:10 or more or even 1:15 or more is most preferred. The upper limit for the amount of anti-solvent is not particularly restricted, but for practical reasons it is generally not preferred that the ratio of solvent/anti-solvent is more than 1:100, and usually a ratio of 1:50 or less, or 1:30 or less is preferred.

If a high excess of anti-solvent is used, it is also possible to use a wider range of possible solvents, because with a high excess of anti-solvent more solvents are miscible in the anti-solvent.

All above solvent/anti-solvent ratios relate to the end of the addition of the solvent to the anti-solvent, i.e. when the complete solution of the agomelatine in the solvent has been added to the anti-solvent. All ratios above are v:v ratios.

It is also preferred that the solution of the agomelatine in the solvent is added slowly to the anti-solvent, preferably dropwise. Thus, in a preferred embodiment the solution in which agomelatine is dissolved in a solvent is added dropwise to an excess of anti-solvent. Preferably, the anti-solvent is stirred during the addition of the solution.

The concentration of the agomelatine in the organic solvent is preferably as high as possible, which means that the amount of solvent used to dissolve the agomelatine is as low as possible. Preferred are concentrations of at least 50% of the saturation concentration, more preferred at least 70%, still more preferred at least 90% of the saturation concentration. The upper limit of the concentration of the agomelatine in the organic solvent is generally the saturation concentration or slightly below the saturation concentration (e.g. 5% below the solution concentration).

When the solution containing the agomelatine dissolved in a solvent has been added to the anti-solvent, agomelatine precipitates. It is not necessary to wait for a certain amount of time after addition of the dissolved agomelatine to the anti-solvent has been completed, however, it is possible to wait for some additional time to allow the crystallization to complete, e.g. stir the mixture for an additional half an hour or hour. However, this is not absolutely necessary for successfully carrying out the process of the invention.

The precipitated crystals of agomelatine are then isolated usually by filtration, but, of course, other isolation methods such as centrifugation that are known in the art can also be used. The isolated crystals are optionally and preferably dried in a conventional manner, preferably at not too high a temperature of 80°C or below, preferably 70°C or below, such as 50°C. The drying of the isolated crystals can be done under reduced pressure, as is known in the art or under atmospheric pressure. Drying at a temperature range of 40°C to 80°C is carried out for generally at least one hour, usually at least two hours, until the required dryness is achieved. Generally it is not necessary to dry for more than 24 hours. Generally, the drying time is 12 to 14 h at a temperature in the range of 45 to 50°C, optionally under reduced pressure.

The crystals of polymorph form I of agomelatine obtained as described above can then be mixed with pharmaceutically acceptable excipients and processed into a pharmaceutical composition (medicament) in a manner known per se. Preferred are tablets, and suitable formulations for tablets are disclosed e.g. in US 2005/0182276. Corresponding examples are disclosed below.

Of course, it is also possible to mix the polymorph form I of agomelatine obtained by the above process with pharmaceutically acceptable ingredients and process it to other medicaments such as capsules, pellets, sachets, etc. The polymorph form I of agomelatine is not particularly susceptible to change to another polymorph form, and therefore, no particular care must be taken when preparing the pharmaceutical composition.

Preferably, the pharmaceutical compositions of the present invention are free from other polymorph forms of agomelatine and contain only the polymorph form I of agomelatine.

By the novel process for preparing agomelatine of polymorph form I a product is obtained which is structurally different from the agomelatine of polymorph form I which is obtained by other methods. In particular, the agomelatine of polymorph form I that has been prepared by the process of the present invention has a very low hygroscopicity, and it is believed that the reason for this very low hygroscopicity is the result of a specific surface of the obtained product particles.

Furthermore, it has been found that the process of the present invention provides crystals with a very narrow particle size distribution. The particle size distribution can be determined by well-known methods, e.g. by optical methods, such as with a Malvern Matersizer 2000.

In particular, the measurement of the particle size distribution can be done by laser diffractometry using a Malvern Mastersizer 2000 Model No. Hydro 2000 S. Preferred is measurement in suspension with dispersion of particles in water (about 55 mg of sample to 10 ml of milli-Q water), a stir rate of 2000 rpm, ultrasound 60 s with obscuration of 9 to 16%. The analysis is carried out for particles with D₅₀ value < 5 µm by Mie method and for particles with D₅₀ value of at least 5 µm by Fraunhofer method.

Of course, other well-known methods and equipment can also be used for determining the particle size and the particle size distribution.

A narrow particle size distribution is highly advantageous for a pharmaceutically active ingredient, since any medicament must have a uniform distribution of the active ingredient and a uniform and highly reproducible bioavailability. Therefore, generally particles are milled and sieved to a certain narrow particle size distribution before processing the particles of an active ingredient into a medicament. With the process of the present invention the particles of the agomelatine of polymorph form I are obtained in a narrow particles size distribution, so that usually no milling and sieving is required or milling and sieving steps can be significantly reduced.

Preferably, the crystals of polymorph form I of agomelatine obtained by the process of the present invention have a D₉₀ value of D₉₀ < 100 µm, more preferably < 50 µm, more preferably < 40 µm, most preferably < 30 µm.

Preferably, the crystals of polymorph form I of agomelatine obtained by the process of the present invention have a D₅₀ value of D₅₀ < 50 µpm, preferably < 30 µm, more preferably < 20 µm, most preferably < 10 µm.

The crystals of the polymorph form I of agomelatine obtained by the process of the present invention preferably have a D₁₀ value of D₁₀ < 30 µm, more preferably < 10 µm, more preferably < 5 µm and most preferably < 2 µm.

Furthermore, the crystals of polymorph form I of agomelatine that can be obtained by the process of the present invention have a narrow particle size distribution, and the particle size distribution is narrower than the particle size distribution of the crystals of agomelatine of polymorph form I that is obtainable by the prior art processes. Usually, the particle size distribution (D₉₀-D₁₀)/D₅₀ is ≤ 1.2, more preferably ≤ 1.0, still more preferably ≤ 0.75 and most preferably ≤ 0.5.

The definition of D₉₀, D₁₀ and D₅₀ is well-known in the art. In particular, the D₉₀ value is the particle size at which 90% of the volume of the particles have a smaller particle size than the D₉₀ value. Accordingly, the D₅₀ value is the particle size at which 50% of the volume of the particles have a smaller particle size than the D₅₀ value. Accordingly, the D₁₀ value is the particle size at which 10% of the volume of the particles have a smaller particle size than the D₁₀ value.

The crystals of polymorph form I of agomelatine of the above very narrow particle size distribution are obtainable by the process of the present application and their particle size distribution is different from the particle size distribution of the crystals of polymorph form I of agomelatine obtained by prior art processes, which is much broader. In particular, the particle size distribution (D₉₀-D₁₀)/D₅₀ of crystals of agomelatine of form I obtainable by the prior art processes is at least 1.3. Thus, the crystals of polymorph form I of agomelatine obtainable by the process of the present invention are distinguishable from the crystals of polymorph form I of agomelatine obtainable by the prior art processes and therefore novel.

The present application also relates to this novel product. The present invention also relates to pharmaceutical compositions that have been prepared using this novel polymorph form I of agomelatine as defined above.

The following examples further explain the present invention, however, the examples are not restrictive.

### Examples

### Example 1:

500 mg of agomelatine were dissolved under stirring at 0°C in 2.5 ml of acetone. This solution was added dropwise to 40 ml of n-heptane at 0°C. Crystallization started immediately, and after the addition of the solution of the agomelatine to the n-heptane was complete, the precipitated crystals were immediately collected by filtration and dried 12 hours at 50°C.

The XRPD of the product was prepared, and it was identical to the XRPD of the known polymorph I of agomelatine within the error margin. The XRPD is shown in figure 1.

### Example 2:

500 mg of agomelatine were dissolved under stirring at 0°C in 2.5 ml of acetone. This solution was added dropwise to 40 ml of n-heptane at -20°C. Crystallization started immediately, and after the addition of the solution of the agomelatine to the n-heptane was complete, the precipitated crystals were immediately collected by filtration and dried 12 hours at 50°C.

### Example 3:

500 mg of agomelatine were dissolved under stirring at 0°C in 2.5 ml of ethanol. This solution is added dropwise to 40 ml of n-heptane at -78°C. Crystallization started immediately, and after the addition of the solution of the agomelatine to the n-heptane was complete, the precipitated crystals were immediately collected by filtration and dried 12 hours at 50°C.

### Example 4:

500 mg of agomelatine was dissolved under stirring at 0°C in 2.5 ml of methanol. This solution is added dropwise to 40 ml of triethylamine at -20°C. Crystallization started immediately, and after the addition of the solution of the agomelatine to the triethylamine was complete, the precipitated crystals were immediately collected by filtration and dried 12 hours at 50°C.

### Example 5:

500 mg of agomelatine were dissolved under stirring at 0°C in 2.5 ml of acetone. This solution was added dropwise to 40 ml of triethylamine at -78°C. Crystallization started immediately, and after the addition of the solution of the agomelatine to the triethylamine was complete, the precipitated crystals were immediately collected by filtration and dried 12 hours at 50°C.

### Example 6:

500 mg of agomelatine were dissolved under stirring at 0°C in 2.5 ml of ethanol. This solution was added dropwise to 40 ml of diisopropylic ether at -20°C. Crystallization started immediately, and after the addition of the solution of the agomelatine to the diisopropylic ether was complete, the precipitated crystals were immediately collected by filtration and dried 12 hours at 50°C.

### Example 7:

500 mg of agomelatine were dissolved under stirring at 0°C in 2.5 ml of acetone. This solution was added dropwise to 40 ml of diisopropylic ether at -78°C. Crystallization started immediately, and after the addition of the solution of the agomelatine to the diisopropylic ether was complete, the precipitated crystals were immediately collected by filtration and dried 12 hours at 50°C.

### Example 8: Pharmaceutical composition

| | |
|---|---|
| Agomelatine form I of example 1 | 25 mg |
| Lactose monohydrate | 123 mg |
| Mg-Stearate | 2 mg |

The above components were mixed and filled into a capsule to provide a capsule containing a dose of 25 mg of agomelatine form I.

### Example 9: Pharmaceutical composition

| | |
|---|---|
| Agomelatine form I of example 1 | 25 g |
| Lactose monohydrate | 62 g |
| Mg-Stearate | 1.3 g |
| Maize starch | 26 g |
| Maltodextrins | 9 g |
| Silica colloidal anhydrous | 0.3 g |
| Sodium starch glycolate type A | 4 g |
| Stearic acid | 2.6 g |

The above components were thoroughly mixed and pressed to 1000 tablets, each containing a dose of 25 mg in a standard tabletting machine.

## Claims

1. Process for the preparation of the polymorph from I of agomelatine, **characterized by** the following crystal data:
C₁₅H₁₇NO₂
Mr = 243.30
Orthorhombic
Pca2₁
a = 31.501 (4) Å
b = 9.5280 (10) A
c = 17.906 (2) Å
Z = 16
the process comprising the process steps of:
a) dissolving agomelatine in a solvent to obtain a solution,
b) adding the solution to an organic anti-solvent having a temperature of 30°C or below, whereby crystals of the polymorph form I of agomelatine are formed, wherein the solvent of step a) is miscible with the anti-solvent of step b),
c) isolating the crystals of the polymorph form I of agomelatine and
d) optionally drying the isolated crystals of the polymorph form I of agomelatine.

2. Process for the preparation of the polymorph form I of agomelatine according to claim 1, wherein in step b) the anti-solvent has a temperature of 20°C or below.

3. Process for the preparation of the polymorph form I of agomelatine according to claim 2, wherein the temperature is in the range of -5°C to 5°C.

4. Process for the preparation of the polymorph form I of agomelatine according to any of claims 1 to 3, wherein the solvent in step a) is selected from the group consisting of methanol, ethanol, propan-2-ol, acetic acid, propylamine, acetone, 1,4-dioxan, dimethylsulfoxide, N-methylpyrrolidone, tetrahydrofuran, ethylacetate and mixtures thereof.

5. Process for the preparation of the polymorph form I of agomelatine according to any of claims 1 to 4, wherein the solution obtained in step a) has a concentration of agomelatine in the range of 90% to 100% of the solution solubility.

6. Process for the preparation of the polymorph form I of agomelatine according to any of claims 1 to 5, wherein step b) is carried out by dropwise addition of the solution to an excess of the anti-solvent.

7. Process for the preparation of the polymorph form I of agomelatine according to any of claims 1 to 6, wherein the anti-solvent in step b) is selected from the group consisting of petrolether, alkanes which are liquid in the temperature range at which step b) is performed, diisopropylether, glycerol, triethylamine and mixtures thereof.

8. Process for the preparation of the polymorph form I of agomelatine according to any of claims 1 to 7, wherein the solvent/anti-solvent ratio at the end of the addition of the solution to the anti-solvent in step b) is at least 1:5 V/V.

9. Process for the preparation of a medicament containing as active ingredient the polymorph form I of agomelatine as defined in claim 1, comprising the following steps:
i) carrying out a process as defined in any of claims 1 to 8 in order to obtain the polymorph form I of agomelatine as defined in claim 1 and
ii) mixing the polymorph form I of agomelatine obtained in step i) with one or more pharmaceutically acceptable excipients.

## Patentansprüche

1. Verfahren zur Herstellung der Polymorph-Form I von Agomelatin, **gekennzeichnet durch** die folgenden Kristalldaten:
C₁₅H₁₇NO₂
Mr = 243,30
Orthorhombisch
Pca2₁
a = 31,501 (4) Å
b = 9,5280 (10) Å
c = 17,906 (2) Å
Z = 16,
wobei das Verfahren folgende Verfahrensschritte umfasst:
a) Lösen von Agomelatin in einem Lösungsmittel zum Erhalt einer Lösung,
b) Zugeben eines organischen Anti-Lösungsmittels mit einer Temperatur von 30°C oder weniger zu der Lösung, wodurch Kristalle der Polymorph-Form I von Agomelatin gebildet werden, wobei das Lösungsmittel von Schritt a) mit dem Anti-Lösungsmittel von Schritt b) mischbar ist,
c) Isolieren der Kristalle der Polymorph-Form I von Agomelatin und
d) gegebenenfalls Trocknen der isolierten Kristalle der Polymorph-Form I von Agomelatin.

2. Verfahren zur Herstellung der Polymorph-Form I von Agomelatin nach Anspruch 1, wobei in Schritt b) das Anti-Lösungsmittel eine Temperatur von 20°C oder weniger aufweist.

3. Verfahren zur Herstellung der Polymorph-Form I von Agomelatin nach Anspruch 2, wobei die Temperatur im Bereich von -5°C bis 5°C liegt.

4. Verfahren zur Herstellung der Polymorph-Form I von Agomelatin nach einem der Ansprüche 1 bis 3, wobei das Lösungsmittel in Schritt a) aus der Gruppe bestehend aus Methanol, Ethanol, Propan-2-ol, Essigsäure, Propylamin, Aceton, 1,4-Dioxan, Dimethylsulfoxid, N-Methylpyrrolidon, Tetrahydrofuran, Essigsäureethylester und Mischungen davon ausgewählt wird.

5. Verfahren zur Herstellung der Polymorph-Form I von Agomelatin nach einem der Ansprüche 1 bis 4, wobei die in Schritt a) erhaltene Lösung eine Agomelatin-Konzentration im Bereich von 90% bis 100% der Lösungslöslichkeit aufweist.

6. Verfahren zur Herstellung der Polymorph-Form I von Agomelatin nach einem der Ansprüche 1 bis 5, wobei Schritt b) durch Zutropfen der Lösung zu einem Überschuss des Anti-Lösungsmittels durchgeführt wird.

7. Verfahren zur Herstellung der Polymorph-Form I von Agomelatin nach einem der Ansprüche 1 bis 6, wobei das Anti-Lösungsmittel in Schritt b) aus der Gruppe bestehend aus Petrolether, Alkanen, die in dem Temperaturbereich, bei dem Schritt b) durchgeführt wird, flüssig sind, Diisopropylether, Glycerin, Triethylamin und Mischungen davon ausgewählt wird.

8. Verfahren zur Herstellung der Polymorph-Form I von Agomelatin nach einem der Ansprüche 1 bis 7, wobei das Lösungsmittel/Anti-Lösungsmittel-Verhältnis am Ende der Zugabe der Lösung zu dem AntiLösungsmittel in Schritt b) mindestens 1:5 V/V beträgt.

9. Verfahren zur Herstellung eines Medikaments, das als Wirkstoff die Polymorph-Form I von Agomelatin gemäß Anspruch 1 enthält, wobei das Verfahren folgende Schritte umfasst:
i) Durchführen eines Verfahrens gemäß einem der Ansprüche 1 bis 8 zum Erhalt der Polymorph-Form I von Agomelatin gemäß Anspruch 1 und
ii) Mischen der in Schritt i) erhaltenen Polymorph-Form I von Agomelatin mit einem oder mehreren pharmazeutisch unbedenklichen Hilfsstoffen.

## Revendications

1. Procédé de préparation de la forme polymorphique I de l'agomélatine, **caractérisée par** les données cristallines suivantes :
C₁₅H₁₇NO₂
Mr = 243,30
Orthorhombique
Pca2₁
a = 31,501 (4) Å
b = 9,5280 (10) Å
c = 17,906 (2) Å
Z = 16
le procédé comprenant les étapes de procédé suivantes :
a) dissolution de l'agomélatine dans un solvant pour obtenir une solution,
b) ajout de la solution à un antisolvant organique présentant une température de 30 °C ou moins, ce qui permet de former les cristaux de la forme polymorphique I de l'agomélatine, où le solvant de l'étape a) est miscible à l'antisolvant de l'étape b),
c) isolement des cristaux de la forme polymorphique I de l'agomélatine et
d) éventuellement séchage des cristaux isolés de la forme polymorphique I de l'agomélatine.

2. Procédé de préparation de la forme polymorphique I de l'agomélatine selon la revendication 1, où dans l'étape b), l'antisolvant présente une température de 20 °C ou moins.

3. Procédé de préparation de la forme polymorphique I de l'agomélatine selon la revendication 2, où la température est dans l'intervalle allant de -5 °C à 5 °C.

4. Procédé de préparation de la forme polymorphique I de l'agomélatine selon l'une quelconque des revendications 1 à 3, où le solvant de l'étape a) est choisi dans le groupe constitué par les suivants : méthanol, éthanol, propan-2-ol, acide acétique, propylamine, acétone, 1,4-dioxane, diméthylsulfoxyde, N-méthylpyrrolidone, tétrahydrofurane, acétate d'éthyle et leurs mélanges.

5. Procédé de préparation de la forme polymorphique I de l'agomélatine selon l'une quelconque des revendications 1 à 4, où la solution obtenue dans l'étape a) présente une concentration en agomélatine dans l'intervalle allant de 90 % à 100 % de la solubilité de la solution.

6. Procédé de préparation de la forme polymorphique I de l'agomélatine selon l'une quelconque des revendications 1 à 5, où l'étape b) est mise en oeuvre par addition au goutte-à-goutte de la solution à un excès de l'antisolvant.

7. Procédé de préparation de la forme polymorphique I d'agomélatine selon l'une quelconque des revendications 1 à 6, où l'antisolvant de l'étape b) est choisi dans le groupe constitué par les suivants : éther de pétrole, alcanes qui sont liquides dans l'intervalle de températures dans lequel l'étape b) est mise en oeuvre, diisopropyléther, glycérol, triéthylamine et leurs mélanges.

8. Procédé de préparation de la forme polymorphique I de l'agomélatine selon l'une quelconque des revendications 1 à 7, où le rapport solvant/antisolvant à la fin de l'addition de la solution à l'antisolvant de l'étape b) est d'au moins 1:5 en volume.

9. Procédé d'élaboration d'un médicament contenant au titre de principe actif la forme polymorphique I de l'agomélatine selon la revendication 1, comprenant les étapes suivantes :
i) mise en oeuvre d'un procédé selon l'une quelconque des revendications 1 à 8 pour obtenir la forme polymorphique I de l'agomélatine selon la revendication 1 et
ii) mélangeage de la forme polymorphique I de l'agomélatine obtenue dans l'étape i) avec un ou plusieurs excipients pharmaceutiquement acceptables.
